# EUROPEAN PATENT SPECIFICATION

(11) **EP 1 677 774 B1**
(45) Date of publication and mention of the grant of the patent: **23.02.2011**
(21) Application number: 04791899.0
(22) Date of filing: 14.10.2004
(51) Int. Cl.: A61K 31/07, A61K 31/4415, A61K 31/355, A61K 31/375, A61P 27/00

(54) **CITICOLINE-BASED COMPOSITION IN COMBINATION WITH VITAMINS FOR THE PREVENTION AND TREATMENT OF EYE PATHOLOGIES**
ZUSAMMENSETZUNG AUF CITICOLIN-BASIS IN KOMBINATION MIT VITAMINEN ZUR PRÄVENTION UND BEHANDLUNG VON AUGENERKRANKUNGEN
COMPOSITION A BASE DE CITICOLINE EN ASSOCIATION AVEC DES VITAMINES POUR LA PREVENTION ET LE TRAITEMENT DE PATHOLOGIES DE L'OEIL

(30) Priority: 22.10.2003 IT RM20030485
(43) Date of publication of application: 12.07.2006
(73) Proprietor: Bausch & Lomb IOM S.p.A., 20050 Macherio (MI) (IT)
(72) Inventor: BAROZZI, Chiara, 00040 POMEZIA (ROME) (IT)
(74) Representative: Banchetti, Marina
(86) International application number: PCT/IT2004/000565
(87) International publication number: WO 2005/037262

(56) References cited:
- EP-A- 1 329 217
- US-A1- 2001 009 678
- US-A1- 2002 099 100
- US-A1- 2002 164 388
- US-A1- 2003 108 624
- US-A1- 2004 180 085
- DATABASE MEDLINE [Online] US NATIONAL LIBRARY OF MEDICINE (NLM), BETHESDA, MD, US; March 2000 (2000-03), CHATZISTEFANOU K I ET AL: "The role of drug treatment in children with strabismus and amblyopia." XP002319128 Database accession no. NLM10937461 & PAEDIATRIC DRUGS. 2000 MAR-APR, vol. 2, no. 2, March 2000 (2000-03), pages 91-100, ISSN: 1174-5878
- REJDAK ROBERT ET AL: "Oral citicoline treatment improves visual pathway function in glaucoma." MEDICAL SCIENCE MONITOR : INTERNATIONAL MEDICAL JOURNAL OF EXPERIMENTAL AND CLINICAL RESEARCH. MAR 2003, vol. 9, no. 3, March 2003 (2003-03), pages PI24-PI28, XP002319127 ISSN: 1234-1010

## Description

The present invention concerns a citicoline-based composition in combination with vitamins for the prevention and treatment of eye pathologies. More specifically, the invention concerns pharmaceutical and/or nutritional compositions based on a choline donor compound, preferably consisting of citicoline, in combination with vitamin A and vitamin B₆, which are effective, also following oral administration, in preserving and restoring the proper structural and functional characteristics of eye tissues in degenerative and non-degenerative ophthalmic pathologies, such as glaucoma and amblyopia, respectively.

As is known, the eye is a hollow, fluid-filled organ divided into two chambers by the crystalline lens. The anterior chamber is bounded at the front by the cornea and on the back by the iris-body ciliary-lens complex. The resulting cavity is full of a transparent liquid, the aqueous humour, produced by ciliary processes. The posterior chamber, which accounts for four-fifths of the volume of the whole eyeball, is bounded at the front by the lens and at the back by the sclera-choroid-retina complex. The resulting cavity is in turn full of a transparent gel, the vitreous humour.

The retina is the nervous tissue which converts luminous stimuli into electric signals. It has a complex structure made up of photoreceptors, bipolar cells, support cells and ganglion cells: The axons of the latter cells line the inner surface of the retina and are bundled together into a sheath, the optic nerve, which serves to convey electric signals to the brain's visual areas (geniculate bodies and visual cortex). The optic nerve is composed of about a million axons and stems from the eyeball at the level of the optic papilla. For its anatomical structural features and complex vascular system, this is a particularly vulnerable region which can be damaged by pathologies, such as eye pressure above physiological limits or systemic or localised haemody-pressure above physiological limits or systemic or localised haemodynamic alterations.

One of the clinically more important ophthalmic pathologies affecting the optic nerve is, as is known, glaucoma, which by itself accounts for about 15% of all cases of blindness in the elderly. It is an insidious disease that in most cases develops slowly and without the patient being aware of the gradual worsening of his/her vision.

In most cases, glaucoma is accompanied by a continuous increase in intraocular pressure which develops following an obstacle to the flow of the aqueous humour and the resulting accumulation of liquid. After a while there is a compression or squeezing of the optic nerve, with the resulting damage or death of the nerve fibres. Damage to the optic nerve leads to a gradual alteration of the visual field, which tends to gradually narrow until it finally disappears. If the optic nerve fibres are damaged, then the visual field will start to have areas where vision is no longer possible, referred to as scotomas, which are initially very small and only affect the outer edges of the visual field. In this situation, the patient continues to see clearly in the centre of the field and thus this damage is often only noticed when the optic nerve damage becomes considerable. When the nerve cells are completely destroyed, the loss of one's sight is final and irreversible.

There are some risk factors which can increase the likelihood of developing the disease, such as old age, eye traumas, hereditary factors, systemic artery hypotension, vascular pathologies, haematic dyscrasia, ischaemic cardiopathologies, prolonged therapy with local or systemic steroid drugs, and severe myopia.

The glaucoma comes about in two different forms as regards onset and extension: a so-called open angle glaucoma and a so-called narrow angle glaucoma. The first type, the primary open angle glaucoma, is - for its epidemiological features and asymptomatic course - the most frequent and insidious form. It is a slow but progressive degenerative neuropathy characterised by intraocular pressure that increases gradually, without there being any particular symptoms. The persistent increase in pressure progressively damages the optic nerve, leading to a narrowing of the visual field and, finally, to blindness, both being irreversible states.

Drug treatment is usually via topical administration, except for the carbonic anhydrase inhibitors, which can also be orally administered. The drug is locally administered as an eye-drop. Drugs can only prevent glaucoma and slow down its development: thus, treatment must start as early as possible and must never be interrupted. The active ingredients used belong to the classes of beta-blockers, prostaglandins, adrenergic agonists, carbonic anhydrase inhibitors and cholinergic agonists.

As already noted, glaucoma is a chronic neurodegenerative disease in which there is a selective death of the retinal ganglion cells (RGC) along with structural changes of the optic nerve head. A series of experimental evidence suggests that in a glaucomatous eye, ganglion cell death is by apoptosis (programmed cell death) caused by the lack of trophic factors to the cell or by retinal ischaemic states. Other factors involved in the selective death of RGCs could be cytotoxic substances produced by the body itself.

Regardless of the cause and mechanism of action leading to RGC death, cell degeneration takes years and there is thus the time available for neuroprotective drug therapy to prevent RGC death. Therefore, the aim of a neuroprotective treatment, which must be complementary to and not replace the eye-drops reducing ocular pressure, is that of directly acting on the progressive degeneration of the cells and of the nerve fibres conveying electric impulses from the retina to the brain, by making them more resistant to various damaging stimuli, recovering them from a suffering state and stimulating their function.

Another eye pathology requiring treatment as early as possible to guarantee some effect is amblyopia. This may be defined as a visual deficit in one or, more rarely, both eyes that is not explained by pathologies identifiable by the oculist, and which cannot be ascribed to organic lesion ascertainable via a physical examination of the eye. Usually, an eye suffering from amblyopia is called a "lazy eye" for the very reason that it does not present a specific pathology, but that its function has not developed completely. More recently, the expression has gradually been taken to mean unilateral visual deficits in patients with strabismus and anisometropia.

Amblyopia can only occur during the period in which the visual apparatus develops: indeed, if the very causes which normally determine it in the first 7-8 years of life intervene when the visual system has fully developed, they can no longer cause it. Moreover, the earlier that one of these causes acts, the more difficult it is to recover proper sight.

The possible causes of amblyopia lie in any factor reducing the visual stimulus in only one eye, such as with anisometropia (difference of a refractive defect between the two eyes), strabismus, the sensorial deprivation caused by any anatomical obstacle or eye pathology that does not allow a normal visual stimulus in one of the two eyes.

Amblyopia treatment, which must start as early as possible for it to have any appreciable therapeutic results, is mainly carried out through two types of interventions, both being necessary to obtain a sufficient visual recovery:
- the identification and removal of the causes thereof, such as visual deprivation factors (congenital cataract, palpebral ptosis, etc.), refraction defects and strabismus;
- rehabilitative treatment with various methods, such as reducing the visual stimulus in the focusing eye and direct stimulation of the amblyopic eye with special devices, or by administering substances that can activate nerve conduction along the optic nerve.

Attempts at devising a rehabilitative treatment for amblyopia with pharmaceutical methods have not been satisfactory so far. In particular, a temporary beneficial effect of L-dopa on some visual functional parameters of amblyopic eyes has been demonstrated, but with non-negligible side effects.

One substance put forward in the scientific literature as a potential candidate for drug therapy useful for the prevention and treatment of eye pathologies characterised by impairment of neuronal activity, such as glaucoma and amblyopia, is cytidine-5'-diphosphocholine, cytidine diphosphate ester of choline, also known as CDP-choline or citicoline, the latter being the international non-proprietary name (Secades, J. J., CDP-choline: update and review of its pharmacology and clinical use, Methods Find. Exp. Clin. Pharmacol. 24 (Suppl.B) (2002), 1-53). CDP-choline is a mononucleotide of natural origin and composed of ribose, cytosine, pyrophosphate and choline, formed starting from phosphocholine and cytidine-5-triphosphate in a reversible reaction catalysed by CTD-choline transferase (CT). Phosphocholine is in turn obtained by phosphorylation of choline, and cytidine-5-triphosphate is obtained by phosphorylation of cytidine.

The biological importance of CDP-choline lies in the fact that it is used by cells as an intermediate of the synthesis of phosphatidylcholines, a group of compounds including many molecular species of phospholipids and, particularly in brain tissue, accounts for over 50% of all cell phospholipids. The latter are not only fundamental structural components of cell membranes, but also act as an important source of free fatty acids, such as arachidonic acid and diacylglycerol, which act as intracell messengers, particularly as transducers of the luminous signal in the retina. Phosphatidylcholine synthesis is regulated by CT and depends on the contribution of choline and cytidine outside the cell.

It is held, however, that a massive degradation of phospholipids can lead to an accumulation of fatty acids and contribute to apoptosis. This mechanism could also concern RGCs in glaucomatous subjects, particularly in old age, since it has been found that as people get older there is an accumulation of free fatty polyunsaturated acids in the retina.

As regards the pharmacokinetics of CDP-choline, a series of tests carried out with radioisotope labelling of the compound demonstrated that this substance, following intravenous or oral administration, is immediately hydrolysed and converted into the two circulating metabolites cytidine and choline. The pharmacokinetic tests also indicate that the calculated bioavailability for oral administration is virtually the same as the one calculated for intravenous administration. The administration of CDP-choline leads to a significant increase in the two metabolites, choline and cytidine, in the blood.

From the scientific literature and through clinical and commercial applications, it is already known that citicoline administration, by increasing the formation of phosphatidylcholine in brain tissue cells while at the same time preventing the accumulation of free fatty acids, leads to an anti-apoptotic and neuroprotective effect. This effect is thought to be mainly carried out by stabilising the membrane, repairing the damaged membrane and via the contribution of substrates that are important for nerve cell functional activity. For this reason, citicoline-based preparations - possibly as the corresponding sodium salt - are already employed, mainly for parenteral administration (intramuscular or intravenous injection, or by infusion), for the treatment of cognitive disorders following brain traumas or damage, cerebral vascular pathologies, Parkinson's disease and generally for the treatment of neuronal disorders of ischaemic, traumatic or degenerative origin.

For these therapeutic indications the possibility has also been put forward, mostly in patent literature, to formulate citicoline with additional substances making the resulting pharmaceutical preparation suitable for oral administration. An example of these formulations is described in US patent No. 4386077, referring to a pharmaceutical composition for oral use based on citicoline, or one of its salts, in association with a substance of the phospholipid group, such as lecithin. Another example is described in US patent application No. 2003/0114415, where, for the same aforesaid therapeutic indications, a citicoline-based composition is proposed, preferably in association with linoleic acid or linolenic acid, or their active metabolites. Here, too, the preferred form of administration proposed is the oral form. Alternatively to citicoline, the document also proposes the use of active metabolites of choline, cytidine and/or uridine.

As regards glaucoma, in particular, long-term clinical studies carried out through the intramuscular administration of CDP-choline have demonstrated - through perimetric measures - that this substance has a positive effect on the damaged optic nerve. Moreover, a short-term clinical study demonstrated that the intramuscular administration of CDP-choline leads to a functional improvement in glaucomatous patients as regards retinal and supraretinal signal conduction (Parisi, V., Manni, G., Colacino, G., Bucci, M., Cytidine-5'-diphosphocholine (citicoline) improves retinal and cortical responses in patients with glaucoma, Ophthalmology, 106 (1999), 1126-34; Virno, M., Pecori-Giraldi, J., Liguori, A., De Gregorio, F., The protective effect of citicoline on the progression of the perimetric defects in glaucomatous patients (perimetric study with a 10-year follow-up), Acta Ophthalmol. Scand. 78, (2000), 56-7; Grieb, P. e Rejdak, R., Pharmacodynamics of citicoline relevant to the treatment of glaucoma, J. Neurosci. Res., 67 (2002), 143-148).

In the study of possible pharmacological treatments for amblyopia, it has been demonstrated that results are possible with a better safety profile with respect to L-dopa by intramuscular administration of large doses of citicoline (Campos E. C., Schiavi, C., Benedetti, P., Bolzani, R., Porciatti, V., Effect of citicoline on visual acuity in amblyopia: preliminary results. Graefe's Arch. Clin. Exp. Ophthalmol., 223 (1995), 307-312; Campos, E.C., Bolzani, R., Schiavi, C., Baldi, A., Porciatti, V., Cytidine-5'-diphosphocholine enhances the effect of part-time occlusion in amblyopia, Doc. Ophthalmol., 93 (1997), 247-263; Porciatti, V., Schiavi, C., Benedetti, P., Baldi, A., Campos, E.C., Cytidine-5'-diphosphocholine improves visual acuity, contrast sensitivity and visually-evoked potentials of amblyopic subjects, Curr. Eye Res., 17 (1998), 141-8).

On the basis of this state of the art, an object of the present invention is to provide a preparation, preferably for oral administration, that can preserve and restore the proper structural and biological characteristics of eye nerve cells by stimulating specific functional activities, in order to be effective in the prevention and treatment of eye pathologies characterised by impairment of neuronal activity, such as glaucoma and amblyopia. In view of the fact that the preparation in question concerns long-term treatment, there is an evident advantage in using an oral administration allowing the patient to avoid the intervention of specialised personnel for administering the product.

To this end, according to the present invention, a pharmaceutical/nutritional formulation is proposed, fundamentally based on the functional group of agents composed of a choline donor compound, vitamin A and vitamin B₆. This association has proved to be very effective in the prevention and treatment of the ophthalmic pathologies considered, and is dearly supported by a synergic interaction between the three fundamental components: this interaction will be illustrated in more detail below.

According to the previous literature, the preferred and mostly studied choline donor compound is CDP-choline or citicoline, but the possibility of including other alternative products in the formulation is not ruled out: the products efficiently providing the body with the necessary choline for the synthesis of phosphatidylcholine and also used by nerve cells for the synthesis of the neurotransmitter acetylcholine. Other usable precursors are, for example, besides choline itself, lecithines.

The second component of the proposed association, vitamin A, carries out an essential role in the visual function, and particularly in the function of the retina, since it is a component of the system dealing with the light reception and the transformation of the luminous impulse into an electric signal via the photoreceptors (cones and rods). Vitamin A is also essential for the functional and structural integrity of epithelial cells. A vitamin A deficit leads to a decrease in visual acuity, especially in conditions of low light intensity.

Vitamin B₆, also known as pyridoxine, in turn can improve the bioavailability of choline for phosphatidylcholine synthesis.

Besides what is predictable on the basis of the known properties of each of the three substances above, the three basic substances of the preparatio according to the present invention, result in a particularly considerable effectiveness of the corresponding pharmaceutical/nutritional product, since they can act synergically on: (1) the metabolic mechanism, both exogenous and endogenous, for phosphatidylcholine biosynthesis; (2) the efficiency of the visual cycle as regards the specialised cells of the retina; (3) the metabolic mechanism leading to acetylcholine synthesis.

Therefore, the present invention specifically provides a composition for the prevention and treatment of eye pathologies including, as fundamental ingredients, a choline donor compound selected from the group consisting of citicoline, choline and lecithines, vitamin A and vitamin B₆. As already noted, the choline donor compound is preferably, but not necessarily, citicoline.

A study of the possible mechanism of action of the proposed combination firstly evidences a functional action on the metabolic mechanism, both exogenous and endogenous, for the phosphatidylcholine biosynthesis. Indeed, as already noted, citicoline is a particularly efficient donor of exogenous choline. Whatever the administration method, citicoline causes an increase in the choline available for the biosynthetic route which, through the formation of phosphorylcholine, regenerates cytidine diphosphate-choline. The latter, in turn reacting with 1,2-diacylglycerol, leads to the formation of phosphatidylcholine.

Moreover, administration of the citicoline-containing composition enhances the endogenous biosynthetic route of phosphatidylcholine. Also, besides choline, the composition provides vitamin B₆, which intervenes as an essential co-factor in the decarboxylation of phosphatidyl-serine for the synthesis of phosphodilethanolamine. The latter substance is an essential intermediate of the endogenous synthesis of phosphatidylcholine.

Moreover, still considering the possible action mechanism of the proposed composition, it is found that vitamin A (a terms used in the chemical identification of retinol and its esters) plays a fundamental role in the function of the retina. In vision, the form of the functional vitamin in photoreception is retinal. The initial step in photoreception, which, as is known, is carried out by two types of retina cells - cones and rods, is the absorption of light by a chromophore, 11-cis-retinal, linked to a receptor of proteic nature. In the rods, the 11-cis-retinal appears combined with opsin to form rhodopsin. The visual process starts with the absorption of a light photon, followed by the photode-composition of the rhodopsin through a cascade of chemical reactions that finally lead to the isomerisation of 11-cis-retinal and to the dissociation of the opsinic portion. The activated rhodopsin interacts rapidly with a protein called transducin, which stimulates a further series of chemical reactions correlated to the cyclical GMP system that finally lead to the generation of action potentials reaching the brain through the optic nerve.

Therefore, the administration of the composition according to the present invention, containing vitamin A as well as citicoline, makes available the precursors necessary for the chemical reactions correlated to the visual process. Moreover, since vitamin B₆, also contained in the composition, seems to increase the affinity of transducin for the rhodopsin system, it is possible to hypothesise a synergic effect of the components of the composition in improving visual functionality. This positive synergic effect may be carried out because citicoline protects and restores the integrity of the cell membrane in the damaged cells and thus, by restoring proper cell functioning, carries out a synergic action with the other components in improving the conduction of the nervous impulse between the retina and the brain. A recent series of experimental results suggests that, as well as this reparative mechanism, citicoline has a neuroprotective effect thanks to its capacity to inhibit the activation of phospholipase A2 (PLA2) and thus to inhibit the destructive processes caused by it. This inhibiting action on PLA2 is extremely important since it is the basis for a series of actions protecting nerve cell structure, such as:
1) Protecting the content of arachidonic acid (which is released thanks to phospholipase action), phosphatidylcholine and phosphodilethanolamine: the oxidative metabolism of the arachidonic acid released contributes to generating reactive oxygen species (ROS), one of the most important causes of neural degeneration;
2) Protecting cardiolipine (a component of the internal membrane of the mytocondria, essential for conveying electrons, whose loss causes an interruption of the respiratory chain and an increase in ROS at the mytocondria level);
3) Increasing phosphatidylcholine levels;
4) Attenuating lipidic peroxidation.

Moreover, reduction of the PLA2 synthesis also causes a reduction in the release of glutamate by the damaged cells, and thus a reduction of its cytotoxic effect. This effect (excitotoxicity of glutamate) is considered to be one of the most likely causes of cell death in glaucomatous neurodegeneration and thus the capacity of citicoline to reduce glutamate release by inhibiting PLA2 activation is a further important mechanism in the neuroprotective function of citicoline. Some experimental results suggest that vitamin A, contained in the composition, can have a synergic action with the choline in inhibiting PLA2.

As a further element in the study of a possible mechanism of action of the proposed composition, one should consider the effect of the composition itself on the metabolic mechanism leading to acetylcholine synthesis. This neurotransmitter is biosynthesised in the presynaptic portion of the cholinergic synapses, starting from different precursors. The series of chemical reactions that, through choline synthesis starting from serine and methionine, lead to the formation of acetylcholine, can use vitamin B₆ as a co-factor in the decarboxylation. Therefore, the presence of choline and vitamin B₆ in the composition may have a synergic effect in stimulating the formation of acetylcholine, in this way increasing the efficiency of the nerve stimulus conduction system at the cholinergic synapses level.

Although the composition according to the present invention can, in theory, be administered via any administration method, including parenteral forms using preparations for injection, it is evident that the greatest advantage of the proposed composition is the form for oral administration. In this case, the pharmaceutical form may be any of the ones normally used for oral administration, such as capsules, tablets, syrups, powders, granules, and aqueous and non-aqueous solutions or suspensions, the preferred form being granules in monodose sachets.

According to some specific embodiments of the invention, the proposed composition contains, per 100 g of the dry product, from 1 g to 50 g of citicoline, from 0.0001 g to 0.1 g of vitamin A and from 0.001 g to 0.1 g of vitamin B₆.

Besides the three aforesaid fundamental ingredients, the formulation may preferably contain vitamin E, specifically in a quantity from 0.001 g to 0.1 g per 100 g of dry product. Similarly, the composition may include vitamin C, preferably in a quantity from 0.01 g to 2 g per 100 g of dry product.

As is known, vitamin E is the most important defence against the peroxidation processes occurring in the lipid layer of the cell membrane. These processes are the main cause of the integrity loss of the membrane itself, and thus of the cell functionality loss, particularly with nerve cells, that is seen with ageing or upon certain pathologies.

On the other hand, for its known properties of acting as a co-factor in many chemical reactions transferring electrons to reducing enzymes, vitamin C also performs an antioxidant activity, thus contributing to maintaining cell membrane integrity.

Therefore, according to some preferred embodiments of the invention, the proposed composition includes the following components in the following weight percentages, with reference to the dry product:

| | |
|---|---|
| citicoline | 1.0 - 50.0 % |
| vitamin A | 0.0001 - 0.1 % |
| vitamin Be | 0.001 - 0.1 % |
| vitamin E | 0.001 - 0.1 % |
| vitamin C | 0.01 - 2.0 % |

A specific applicative form of the invention envisages a granular preparation containing the following components in the following quantities, per 100 g of powder:

| | |
|---|---|
| citicoline | 9.0 - 14.0 g |
| vitamin A | 3.0 - 4.5 mg |
| vitamin, Be | 4.5 - 7.0 mg |
| vitamin E | 37.00 - 57.0 mg |
| vitamin C | 225.0 - 340.0 mg |

A preferred formulation for the aforesaid preparation is the following one (again with reference to 100 g of dry product):

| | |
|---|---|
| citicoline | 11.40 g |
| vitamin A | 3.77 mg |
| vitamin B₆ | 5.70 mg |
| vitamin E | 47.00 mg |
| vitamin C | 285.00 mg |

The aforesaid preparation includes the said fundamental ingredients together with a mixture of excipients suitable for administration of the product in granular form for oral use. Such preparation can be made according to criteria and with ingredients that are well known in the pharmaceutical art. A preferred example for the choice of excipients is a mixture of inulin, maltodextrin, citric acid, flavours, sweeteners and colorants.

Instead of inulin, other products can obviously be used for the main support of the granulate, for diluting and mixing the components, such as other polysacharides, including starch, cellulose, etc. Instead of, or along with, maltodextrin, one may also include, e.g., lactose, while aspartame, sucrose, acesulfame, fructose and the like can be used as a sweetener. An example of colorant may be a solution of 1 % β-carotene.

The preparation according to the invention may also contain further specific groups of biofactors or biomolecules with well-known useful physiological effects. For example, it is possible to include other antioxidants besides vitamin E and vitamin C, such as the coenzyme Q-10, flavonoids, amino acids with antioxidant activity (such as arginine, taurine, cysteine), resveratrol, ginkgo biloba, alpha-lipoic acid and quercetin. The preparation may also include water-soluble vitamins, such as nicotinic acid and nicotinamide, as essential coenzymes for tissue respiratory processes.

Trace elements essential for one's diet can also be included, such as zinc, selenium, chromium, vanadium, calcium, magnesium and copper, and/or amino acids functioning as precursors of important molecules, such as L-arginine, the precursor of nitric oxide (NO), and/or agents correlated to neuro-transmission, such as: precursors of neurotransmitter synthesis (besides CDP-choline), for example, choline and phosphatidylcholine DMAE; agents stimulating neurotransmitter secretion, such as phosphatidylserine; agents with the capacity to inhibit neurotransmitter degradation, such as inhibitors of acetylcholinesterase; phytoestrogens capable of stimulating the production of enzymes involved in neurotransmitter synthesis.

Other ingredients which may be added to the claimed composition are agents necessary for maintaining cell membrane structure and functions, including such substances as alpha-linolenic acid, docosahexaenoic acid (DHA), phosphatidylserine, phosphatidylethanolamine, phosphatidyl-inositol, S-adenosylmethionine; agents capable of stimulating the production of NO and cyclic guanosin 3',5'-monophosphate (or cGMP), such as L-arginine, N-acetylcysteine, extracts of ginkgo biloba, riboflavin, folic acid; agents involved in maintaining blood levels of glucose, among which some particularly important are, for example, melatonin, nicotinamide and alpha-lipoic acid.

According to a further aspect, the present invention provides the use of a combination of a choline donor compound selected from the group consisting of citicoline, choline and lecithines, vitamin A and vitamin B₆ for the preparation of a pharmaceutical/nutritional product for the prevention and treatment of eye pathologies, particularly when the said choline donor compound is citicoline. Preferably, the said product also includes vitamin E and vitamin C.

In the realisation of an exemplificative product according to the present invention, to be administered orally, the composition with the ingredients in the pre-established proportions is prepared as a dry granulate by a process of powdering, mixing and final granulation. The composition thus obtained is packaged in sachets made of composite material consisting of aluminium, polyethylene and paper.

Preferably, each sachet contains a total of 3.5 g of granular product and, in the case of a composition according to the aforesaid preferred quantitative proportions, the dosage may vary between 1 and 8 sachets per day, while the preferred dosage is 2-4 sachets per day.

As already noted, the product is indicated as a pharmaceutical/nutritional preparation useful for the prevention and treatment of eye pathologies characterised by impairment of neuronal activity, and particularly for the treatment of glaucoma and amblyopia.

Some experimental results obtained within the frame of the present invention, showing the effects of the oral administration of a composition according to the invention to patients suffering from open angle glaucoma, are reported below by way of example. Some of the said experimental results are also shown in the graphs of the accompanying drawings, wherein:
Figure 1 shows the effects of oral administration of the composition according to the invention on contrast sensitivity in glaucoma patients, in comparison with the basal values of contrast sensitivity in control patients, receiving no treatment (mean values);
Figure 2 shows the effects of oral administration of the composition according to the invention on contrast sensitivity in glaucoma patients, in terms of percent increase of contrast sensitivity versus basal values (mean values); and
Figure 3 shows the changes in contrast sensitivity (with a spatial frequency of 12 cycles per degree) in time, after repeated periods of treatment with the composition of the invention (mean values).

### Effects of the composition according to the invention on contrast sensitivity in glaucoma patients

Contrast sensitivity refers to the ability of the visual system to distinguish between an object and its background. Contrast sensitivity testing describes a number of subtle levels of vision, not accounted for by visual acuity test; it can quantify very accurately the loss of vision in different pathologies, including cataracts, neuro-ophthalmic diseases and retinal diseases.

Contrast sensitivity measures luminance between brightly and dimly lit areas. Both the distance between the areas (frequency) and the intensity of the areas (luminance) are adjusted over a range. By this test the lowest contrast level which can be detected by a patient for a given seize target is determined.

As normally a range of target sizes are used, this test measures two variables, that is size and contrast, while visual acuity testing measures only size, and is determined at the very high contrast, that is at the highest visible frequency, thus representing a single point of the contrast sensitivity curve. Contrast sensitivity function increases at low frequency, reaches a maximum at an intermediate frequency and then decreases.

The contrast sensitivity function is an accurate method by which certain disease states may be followed. For example, there is a reduction of contrast sensitivity in patients with light scatter lesions, as it is for example in patients with cataract or corneal oedema. Since the contrast sensitivity is dependent upon central nervous system processing, also pathologies affecting the optic nerve function, like glaucoma, can induce a decrease in contrast sensitivity.

In order to evaluate the effects of the oral administration of the proposed composition on contrast sensitivity in glaucoma patients, a preparation having the following weight percent composition in the active ingredients was employed:

| | |
|---|---|
| citicoline | 11.4 % |
| vitamin A | 0.00377 % |
| vitamin B₆ | 0.0057 % |
| vitamin E | 0.047 % |
| vitamin C | 0.285 % |

Patients were recruited among those suffering from primary open angle glaucoma (POAG), and were were divided into two groups (Control and Test Composition).

### Inclusion criteria:

1. Both genders
2. Age between 40 and 90 y.o.
3. Diagnosis: Primary Open Angle Glaucoma (grading III or IV according to Shaffer's Classification),
4. Pharmacologically normalised IOP (i.e. not greater than 19 mmHg as the peak of the daily curve)
5. Early visual field defects corresponding to an MD index not greater than 3.5 dB
6. Patients well trained and reliable to contrast sensitivity testing

### Exclusion criteria:

1. Ocular malformations
2. Non glaucomatous neuropticopathies
3. History of ocular surgery
4. Glaucoma laser procedures (e.g. argon laser trabeculoplasty)
5. Cornea or lens opacities (as far as the lens transparency is concerned, up to grade N1CtrP0 according to LOCS II was allowed)
6. Juvenilis and congenital glaucoma
7. Uveitis and Possner Schlossman Syndrome
8. Hypersensitivity or allergy to the product ingredients
9. Concomitant treatment with psychotropic drugs
10. Concomitant treatment with intramuscular citicoline or any other drug or substance with neurotrophic/neuroprotective activity (e.g. ginkgo biloba extracts, nicergoline, gangliosides etc.) throughout 6 moths prior the recruitment to the study
11. Cholinergic therapies, both topical and systemic
12. Any topical treatment excluding the eyedrops daily taken to reduce IOP
13. Retinal or cerebral diseases affecting the visual field
14. Any neurological condition interfering with the execution of contrast sensitivity testing (e.g. cognitive and behavioural disorders, Parkinson's disease, Alzheimer's disease, senile dementia, etc.)
15. Poor patient's compliance
16. Drug or alcohol addiction
17. Pregnancy or lactation

### Treatment schedule:

The following treatment schedule was used:
   - 1° Cycle : 3 sachets/day of Test Composition for 45 consecutive days
   - SUSP : Suspension of any treatment for 45 days
   - II° Cycle : 3 sachets/day of Test Composition for 45 consecutive days

As a contrast sensitivity test, contrast vision was evaluated by means of Vistech VCTS 6500 (Vistech Consultant Ltd.)

Statistical significance was evaluated by using a model for non parametric data of ANOVA for repeated measures.

### Results

To evaluate the effects of repeated administration of the Test Composition on contrast sensitivity in glaucoma patients the following experimental schedule was used: 10 subjects were treated with 3 sachets/day of Test Composition for 45 consecutive days (I° cycle). At the end of this period the treatment was suspended for 45 days. Then the patients were treated again for 45 consecutive days (II° cycle) using the same treatment schedule used for the I° cycle.

For each patient contrast sensitivity was determined before treatment, at the end of the first treatment cycle, at the end of the suspension period and at the end of the second cycle of treatment.

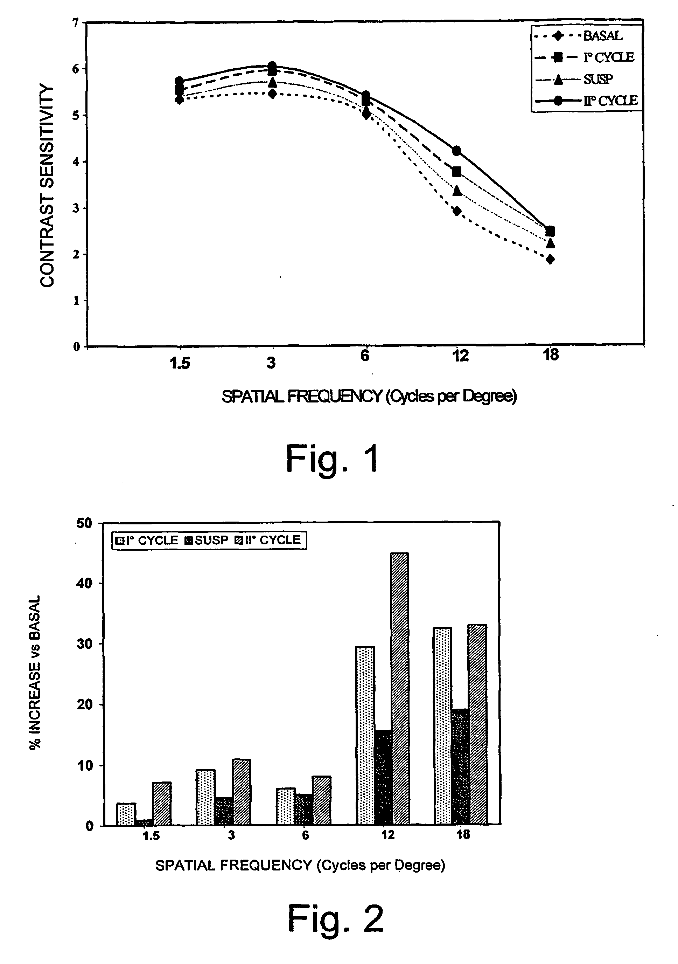

The results, reported in Figure 1, showed that already at the first cycle the administration of the Test Composition induced an increase of contrast sensitivity at all spatial frequencies. Subsequently, at the end of the suspension period the values of contrast sensitivity decreased, although remaining higher than basal values. The second cycle of treatment again induced an increase of contrast sensitivity at all spatial frequencies. Interestingly, the effects observed at the end of the second cycle of treatment were higher than those obtained at the end of the first cycle.

The same results, presented in Figure 2 in terms of percent increase in contrast sensitivity versus basal values, also showed that the effect of the Test Composition is particularly evident when higher spatial frequencies are considered. In fact, both at 12 and 18 cycles per degree, and particularly at 12 cycles per degree, there is a significant percentage increase of contrast sensitivity after the I° and, mainly, after the II° cycle of treatment.

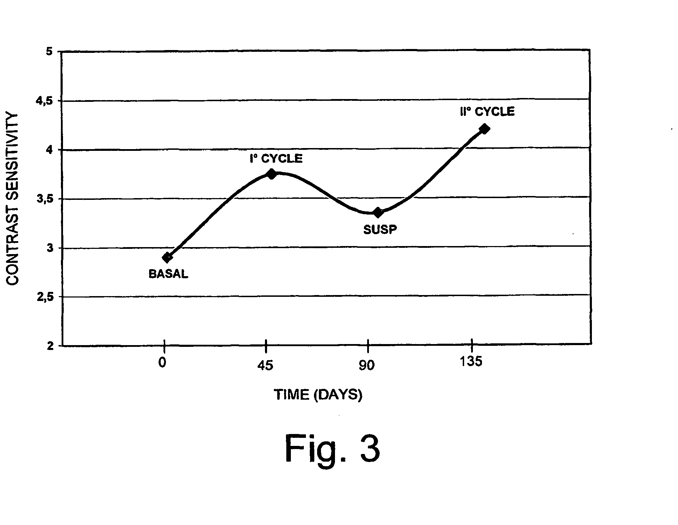

In addition, the results as shown in the graph of Figure 3 indicate that the effect of the administration of the Test Composition decreases-with time after it is discontinued, and that the II°Cycle of treatment induces a higher increase in the response in respect to the increase' obtained after the first cycle.

In view of the foregoing results, it is evident that the administration of the composition according to the invention to glaucoma patients induces an increase in contrast sensitivity, particularly remarkable at medium/high spatial frequencies (i.e. 12 cycles per degree). These frequencies, according to literature, describe the functional status of retinal ganglion cells (RGC). Since RGC represent the main target of the disease and their death leads to optic nerve cupping and to visual field loss, the stimulation and the protection of their function helps in preventing the progression of the disease.

Although contrast sensitivity is not a highly specific test (high frequency of false positives) in glaucoma diagnostics, as many factors can modify the responses to the test, it is very sensitive (low frequency of false negatives) in assessing the early manifestation of RGC function impairment.

Present results indicate that the effect of the administration of the claimed composition on contrast sensitivity tends to decrease with time after it is discontinued, but an additional cycle of treatment induces a higher increase in the response when compared to the increase obtained after the first cycle. Taken together, the results show that repeated administrations can induce a persistent increase in contrast sensitivity, thus suggesting the necessity of periodical cycles of treatment. The present study provides evidence of a persistent neuro-protective effect of the preparation according to the invention and confirm its usefulness as a necessary complement to the standard hypotensive treatment of glaucoma.

The present invention has been disclosed with particular reference to some specific embodiments thereof, but it should be understood that modifications and changes may be made by the persons skilled in the art without departing from the scope of the invention as defined in the appended claims.

## Claims

1. A composition for the prevention and treatment of eye pathologies including, as fundamental ingredients, a choline donor compound selected from the group consisting of citicoline, choline and lecithines, vitamin A and vitamin B₆.

2. A composition according to claim 1, wherein the said choline donor compound is citicoline.

3. A composition according to claims 1 or 2, presented in suitable form for oral administration.

4. A composition according to claim 3, presented in solid form as a granular product.

5. A composition according to claim 4, containing, per 100 g of dry product, from 1 to 50 g of citicoline, from 0.0001 g to 0.1 g of vitamin A and from 0.001 g to 0.1 g of vitamin B₆.

6. A composition according to any one of the aforesaid claims, also containing vitamin E.

7. A composition according to claim 6, containing, per 100 g of dry product, from 0.001 g to 0.1 g of vitamin E.

8. A composition according to any one of the aforesaid claims, also containing vitamin C.

9. A composition according to claim 8, containing, per 100 g of dry product, from 0.01 g to 2 g of vitamin C.

10. A composition according to any one of the aforesaid claims, containing the hollowing components in the following weight percentages, with reference to the dry product.
| | |
|---|---|
| citicoline | 1.0 - 50.0 % |
| vitamin A | 0.0001 - 0.1 % |
| vitamin B₆ | 0.001 - 0.1 % |
| vitamin E | 0.001 - 0.1 % |
| vitamin C | 0.01 - 2.0 % |

11. A composition according to claim 10, in granular form, containing, per 100 g of dry product, the following components in the following quantities:
| | |
|---|---|
| citicoline | 9.0 - 14.0 g |
| vitamin A | 3.0 - 4.5 mg |
| vitamin B₆ | 4.5 - 7.0 mg |
| vitamin E | 37.00 - 57.0 mg |
| vitamin C | 225.0 - 340.0 mg |

12. A composition according to claim 11 having the following formulation, per 100 of dry product:
| | |
|---|---|
| citicoline | 11.40 g |
| vitamin A | 3.77 mg |
| vitamin B₆ | 5.70 mg |
| vitamin E | 47.00 mg |
| vitamin C | 285.00 mg |

13. A composition according to claims 11 or 12, also containing a mixture of excipients composed of inulin, maltodextrin, citric acids, flavours, sweeteners and colorants.

14. Use of a combination of a choline donor compound selected from the group consisting of citicoline, choline and lecithines, vitamin A and vitamin B₆ for the preparation of a pharmaceutical/nutritional product for the prevention and treatment of eye pathologies.

15. Use according to claim 14, wherein the said choline donor compound is citicoline.

16. Use according to claim 15, wherein the said product is indicated for the prevention and treatment of glaucoma.

17. Use according to claim 15, wherein the said product is indicated for the treatment of amblyopia.

18. Use according to claims 14 or 15, wherein the said pharmaceutical/nutritional product also includes vitamin E.

19. Use according to claim 18, wherein the said pharmaceutical/nutritional product also includes vitamin C.

## Patentansprüche

1. Zusammensetzung zur Verhütung und Behandlung von Augenerkrankungen, umfassend als fundamentale Bestandteile eine Cholin-Donorverbindung, ausgewählt aus der Gruppe, welche aus Citicolin, Cholin und Lecithinen besteht, Vitamin A und Vitamin B₆.

2. Zusammensetzung nach Anspruch 1, wobei die Cholin-Donorverbindung Citicolin ist.

3. Zusammensetzung nach Anspruch 1 oder 2, welche in geeigneter Form für die orale Verabreichung dargeboten wird.

4. Zusammensetzung nach Anspruch 3, welche in fester Form als granuliertes Produkt dargeboten wird.

5. Zusammensetzung nach Anspruch 4, welche pro 100 g Trockenprodukt 1 bis 50 g Citicolin, 0,0001 g bis 0,1 g Vitamin A und 0,001 g bis 0,1 g Vitamin B₆ enthält.

6. Zusammensetzung nach irgendeinem der vorgenannten Ansprüche, welche ferner Vitamin E enthält.

7. Zusammensetzung nach Anspruch 6, welche pro 100 g Trockenprodukt 0,001 g bis 0,1 g Vitamin E enthält.

8. Zusammensetzung nach irgendeinem der vorgenannten Ansprüche, welche ferner Vitamin C enthält.

9. Zusammensetzung nach Anspruch 8, welche pro 100 g Trockenprodukt 0,01 g bis 2 g Vitamin C enthält.

10. Zusammensetzung nach irgendeinem der vorgenannten Ansprüche, welche die folgenden Komponenten in den folgenden Gewichtsprozentsätzen, bezogen auf das Trockenprodukt, enthält:
| | |
|---|---|
| Citicolin | 1,0-50,0 % |
| Vitamin A | 0,0001-0,1 % |
| Vitamin B₆ | 0,001-0,1 % |
| Vitamin E | 0,001-0,1 % |
| Vitamin C | 0,01-2,0 %. |

11. Zusammensetzung nach Anspruch 10 in granulierter Form, welche pro 100 g Trockenprodukt die folgenden Komponenten in den folgenden Mengen enthält:
| | |
|---|---|
| Citicolin | 9,0-14,0 g |
| Vitamin A | 3,0-4,5 mg |
| Vitamin B₆ | 4,5-7,0 mg |
| Vitamin E | 37,00-57,0 mg |
| Vitamin C | 225,0-340,0 mg. |

12. Zusammensetzung nach Anspruch 11, welche die folgende Formulierung pro 100 g Trockenprodukt aufweist:
| | |
|---|---|
| Citicolin | 11,40 g |
| Vitamin A | 3,77 mg |
| Vitamin B₆ | 5,70 mg |
| Vitamin E | 47,00 mg |
| Vitamin C | 285,00 mg. |

13. Zusammensetzung nach den Ansprüchen 11 oder 12, welche ferner eine Mischung von Exzipienten, zusammengesetzt aus Inulin, Maltodextrin, Citronensäuren, Geschmacksstoffen, Süßstoffen und Färbemitteln, enthält.

14. Verwendung einer Kombination von einer Cholin-Donorverbindung, ausgewählt aus der Gruppe, welche aus Citicolin, Cholin und Lecithinen besteht, Vitamin A und Vitamin B₆ zur Herstellung eines pharmazeutischen Produkts oder Nahrungsprodukts zur Verhütung und Behandlung von Augenerkrankungen.

15. Verwendung nach Anspruch 14, wobei die Cholin-Donorverbindung Citicolin ist.

16. Verwendung nach Anspruch 15, wobei das Produkt für die Verhütung und Behandlung von Glaukomen indiziert ist.

17. Verwendung nach Anspruch 15, wobei das Produkt für die Behandlung von Amblyopie indiziert ist.

18. Verwendung nach Anspruch 14 oder 15, wobei das pharmazeutische Produkt oder Nahrungsprodukt ferner Vitamin E einschließt.

19. Verwendung nach Anspruch 18, wobei das pharmazeutische Produkt oder Nahrungsprodukt ferner Vitamin C einschließt.

## Revendications

1. Composition destinée à la, prévention et au traitement de pathologies oculaires, comprenant, en tant qu'ingrédients fondamentaux, un composé donneur de choline sélectionné dans le groupe composé de la citicoline, de la choline et des lécithines, de la vitamine A et de la vitamine B₆.

2. Composition selon la revendication 1, dans laquelle ledit composé donneur de choline est la citicoline.

3. Composition selon la revendication 1 ou 2, présentée sous une forme appropriée pour une administration par voie orale.

4. Composition selon la revendication 3, présentée sous une forme solide, en tant que produit granulaire.

5. Composition selon la revendication 4, contenant, pour 100 g de produit sec, 1 à 50 g de citicoline, 0,0001 g à 0,1 g de vitamine A et 0,001 à 0,1 g de vitamine B₆.

6. Composition selon l'une quelconque des revendications précédentes, contenant aussi de la vitamine E.

7. Composition selon la revendication 6, contenant, pour 100 g de produit sec, 0,001 à 0,1g de vitamine E.

8. Composition selon l'une quelconque des revendications précédentes, contentant aussi de la vitamine C.

9. Composition selon la revendication 8, contenant, pour 100 g de produit sec, 0,01g à 2g de vitamine C,

10. Composition selon l'une quelconque des revendications précédentes, contenant les composants suivants, dans les pourcentages en poids suivants, en faisant référence au produit sec.
| | |
|---|---|
| Citicoline | 1,0 - 50,0 % |
| Vitamine A | 0,0001 - 0,1 % |
| Vitamine B₆ | 0,001 - 0,1 % |
| Vitamine E | 0,001 - 0,1 % |
| Vitamine C | 0,01 - 2,0 % |

11. Composition selon la revendication 10, sous forme granulaire, contenant, pour 100 g de produit sec, les composants suivants, dans les quantités suivantes :
| | |
|---|---|
| Citicoline | 9,0 - 14,0 g |
| Vitamine A | 3.0 - 4,5 mg |
| Vitamine B₆ | 4,5 - 7,0 mg |
| Vitamine E | 37,00 - 57,0 mg |
| Vitamine C | 225,0 - 340,0 mg |

12. Composition selon la revendication 11, présentant la formulation suivante, pour 100 g de produit set :
| | |
|---|---|
| Citicoline | 11,40 g |
| Vitamine A | 3,77 mg |
| Vitamine B₆ | 5,70 mg |
| Vitamine E | 47,00 mg |
| Vitamine C | 285,00 mg |

13. Composition selon la revendication 11 ou 12, contenant aussi un mélange d'excipients composés d'inuline, de maitodextrine, d'acides citriques, d'arômes, d'edulcorants et de colorants.

14. Utilisation d'une combinaison d'un composé donneur de choline sélectionné dans le groupe composé de la citicoline, de la choline et des lécithines, de vitamine A et de vitamine B₆ pour la préparation d'un produit pharmaceutique/nutritionnel destiné à la prévention et au traitement de pathologies oculaires.

15. Utilisation selon la revendication 14, dans laquelle ledit composé donneur de choline est la citicoline,

16. Utilisation selon la revendication 15, dans laquelle ledit produit est indiqué pour la prévention et le traitement d'un glaucome.

17. Utilisation selon la revendication 15, dans laquelle ledit produit est indiqué pour le traitement d'une amblyopie,

18. Utilisation selon la revendication 14 ou 15, dans laquelle ledit produit pharmaceutique/nutritionnel contient aussi de la vitamine E.

19. Utilisation selon la revendication 18, dans laquelle ledit produit pharmaceutique/nutritionnel contient aussi de la vitamine C.
